# EUROPEAN PATENT APPLICATION

(11) **EP 4 530 888 A1**
(43) Date of publication of application: **02.04.2025**
(21) Application number: 23200875.5
(22) Date of filing: 29.09.2023
(51) Int. Cl.: G06F 16/906, G06N 3/00

(54) **A DATA PROCESSING SYSTEM AND A DATA-DRIVEN DECISION SUPPORT METHOD FOR HEALTHCARE APPLICATIONS**

(71) Applicant: VITO NV, 2400 Mol (BE)
(72) Inventor: ELEN, Bart, 2400 Mol (BE)
(74) Representative: V.O.

(57) **Abstract**

A data processing system and method for providing data-driven decision support in healthcare applications is provided. The system comprises a primary machine learning model configured to generate predictions based on patient data; an uncertainty module, linked to the primary machine learning model, configured to calculate uncertainty for each prediction of the primary machine learning model; an out-of-distribution (OOD) module, incorporating an OOD detector, wherein the OOD module is configured to assess whether the patient data is in-distribution or out-of-distribution; an auxiliary machine learning model, trained to receive at least inputs from the uncertainty module and OOD module, and based on the inputs to output a value indicative for which cases a predetermined target performance level for predictions generated by the primary machine learning model is met.

## Description

### FIELD OF THE INVENTION

The invention relates to a data processing system for providing data-driven decision support in healthcare applications. Furthermore, the invention relates to a data-driven decision support method for healthcare applications, implemented in a data processing system. Also, the invention relates to a system comprising a plurality of distributed data processing systems according to the invention.

### BACKGROUND TO THE INVENTION

Medical machine learning processing systems are typically evaluated on data from a limited number of clinical practices. This is mostly because it may not be practical or even feasible to obtain data from a large amount of clinical practices. The performance obtained on this data is presented as 'the model performance' (e.g. the model obtains an F1 score of 90%). Some model performance metrics are dependent on the class prevalence and will change when the prevalence changes (e.g. accuracy). Other metrics are not.

Such machine learning products often claim high predictive accuracies based on results obtained from certain populations or datasets. However, these claims are not always substantiated when deployed in different environments/settings or with different datasets. A significant problem arises when these products encounter data of inferior quality or data vastly different from the training set. The current state of art presents an inconsistency in the performance of these machine learning systems across diverse healthcare settings such as medical/clinical practices, potentially resulting in misleading representations of their efficacy.

In the prior art, samples can be taken in hospitals for labeling, with the goal of evaluating the performance of the machine learning processing system within that specific hospital environment. However, this approach relies on inhospital labeling of data and primarily focuses on identifying performance metrics rather than ensuring the sustained accuracy of the machine learning model across different settings.

In the known art, for critical AI applications, human graders can be employed to manually check the predictions of their artificial intelligence (AI) systems. In some cases, users have to wait an extensive period of time for the analysis reports of e.g. the brain MRI image, not because the AI system is slow in performing the prediction, but because the human grader needs time to check the model predictions. Such a solution is very costly, labor intensive and time consuming. There is a need for an improved solution that can be applied to a wide range of medical applications (e.g. screening for COPD on CT images, detecting signs of DR on fundus pictures, or screening for breast cancer on X-ray images), and is able to inform the medical practitioner for each patient examination if the output of the AI can be trusted. For all cases where has been indicated that the AI can be trusted, the medical practitioner should have the guarantee that the AI reaches the promised performance level.

In view of the limitations of existing solutions, there is a strong need for introducing a data processing system and a data processing method that substantially improves the reliability and generalizability of machine learning products in healthcare settings. There is a desire to provide for a robust data processing system wherein the proclaimed accuracy claims of a medical machine learning product can be substantiated and guaranteed for different clinical settings, enhancing the reliability of such products through time and across various healthcare setups.

### SUMMARY OF THE INVENTION

It is an object of the invention to provide for a method and a system that obviates at least one of the above mentioned drawbacks.

Additionally or alternatively, it is an object of the invention to improve a data processing system for providing data-driven decision support in healthcare applications.

Additionally or alternatively, it is an object of the invention to provide for a data processing system for providing data-driven decision support in healthcare applications that can better determine whether predictions generated meet a predetermined target performance level.

Thereto, the invention provides for a data processing system for providing data-driven decision support in healthcare applications, comprising: a primary machine learning model configured to generate predictions based on patient data; an uncertainty module, linked to the primary machine learning model, configured to calculate uncertainty for each prediction of the primary machine learning model; an out-of-distribution (OOD) module, incorporating an OOD detector, wherein the OOD module is configured to assess whether the patient data is in-distribution or out-of-distribution; an auxiliary machine learning model, trained to receive at least inputs from the uncertainty module and OOD module, and based on the inputs to output a value indicative of for which cases a predetermined target performance level for predictions generated by the primary machine learning model is met.

The value can be indicative of whether the prediction of the primary machine learning model can be trusted or not. A predetermined target performance level for predictions generated by the primary machine learning model can be guaranteed for the predictions trusted by the auxiliary machine learning model.

A data processing system is provided that effectively monitors and assesses the predictions generated by the primary model. The system can be used to ensure that the machine learning model maintains its claimed level of accuracy, even when faced with varying data qualities and types. Moreover, the system is equipped to distinguish and communicate the reliability of predictions on data that it was designed for, clearly demarcating it from predictions made on data falling outside its optimized range, thereby avoiding misleading accuracy claims.

The primary machine learning model operates by taking in patient data and generating predictions. Alongside this, there's an uncertainty module that calculates how uncertain each prediction is. The system also has an OOD module containing multiple detectors to verify whether the patient data being processed falls within the expected range (in-distribution) or is anomalous (out-of-distribution). An auxiliary machine learning (ML) model takes inputs from both the uncertainty and OOD modules, then outputs a value indicating if the primary model's prediction reaches a set performance level. The auxiliary machine learning model can thus act as a guaranteed performance model.

By establishing a cohesive and adaptable data processing system that can be deployed across multiple medical settings, the reliability in performance in healthcare machine learning prediction systems can be improved, ensuring that accuracy claims are not just theoretical values but are realized effectively in specific applications. In this way, a more reliable and enhanced integration of machine learning products in the healthcare sector becomes possible.

Optionally, the primary machine learning model has a plurality of outputs.

The OOD Module can be configured to incorporate multiple detectors to discern if the patient data is ID or OOD, facilitating the assessment of data based on its distribution compared to the initial dataset used by the system manufacturer.

Optionally, a calibration module is provided which is communicatively linked to the auxiliary machine learning model, the calibration module being configured to: calibrate the uncertainty outputted by the auxiliary machine learning model, thereby generating an estimated accuracy of predictions from the primary machine learning model; compare the estimated accuracy against the predetermined target performance level threshold; and transmit a signal to an alarm module to activate the alarm signal when the estimated accuracy falls below the predetermined target performance level threshold.

Optionally, the system further comprises an alarm module communicatively linked to the auxiliary machine learning model, configured to assess the value outputted by the auxiliary machine learning model, wherein the alarm module includes a performance monitoring algorithm designed to compare the value against the predetermined target performance level threshold and output an alarm signal via at least one user interface if the value falls below the predetermined target performance level threshold.

The alarm module may be linked to the auxiliary machine learning model and configured to implement a performance monitoring algorithm that assesses the output value from the auxiliary machine learning model against a predetermined threshold. If the value falls below this threshold, an alarm signal is initiated, alerting through a user interface.

In such examples, an alarm module can be connected to the auxiliary machine learning model, and the alarm module continuously monitors the value generated by the auxiliary model. If this value falls below a threshold (i.e., the set performance level), the system raises an alarm signal, alerting the user through a designated interface. The integration of an alarm module facilitates real-time performance monitoring. By continuously comparing the performance level output from the auxiliary machine learning model against a predetermined threshold, it acts as a safeguard against subpar predictions, ensuring immediate response to potential discrepancies and maintaining the system's reliability.

In some examples, first, the uncertainty of the auxiliary model is calibrated so that the accuracy of the primary model can be estimated. When the estimated model accuracy is lower than the threshold value, the alarm can be activated using the alarm module.

Optionally, the employed auxiliary machine learning model is trained using both in-distribution data and synthetic OOD data that is created by applying a plurality of transformations to the in-distribution data used for training and evaluation of the primary machine learning model.

The auxiliary machine learning model, which may be configured to oversee the primary model's performance, can be trained with both standard (in-distribution) data and synthetic out-of-distribution data. This synthetic data is generated by modifying the standard training data to represent unusual or unexpected situations. Advantageously, the robustness of the auxiliary machine learning model can be improved, enhanced by training it with a diverse dataset that includes both in-distribution and synthetic out-of-distribution data. This diverse training dataset prepares the model to handle a broader range of data scenarios and detect even subtle anomalies more effectively.

The synthetic OOD data can be generated through the application of an array of transformations on In-Distribution (ID) data - the data available at the time of Al model creation and evaluation.

This synthetic OOD data serves as an input for the creation of the auxiliary (guaranteed performance) model, which operates in tandem with the primary machine learning model. The auxiliary (guaranteed performance) model is designed to assess and guarantee the claimed performance levels of the machine learning model by analyzing inputs from various modules within the data processing system, including an uncertainty module and the OOD module. It can evaluate whether the prediction generated by the primary machine learning model meets a predetermined target performance level.

In some examples, metrics are used that are specific to individual hospital environments. A data analysis environment may be employed wherein data attributes such as image quality, patient demographics, and others are mapped. The system may be configured to provide predictive analytical curves or surfaces, which encapsulate the expected operational accuracy of the machine learning model across diverse data attributes. These curves, serving as performance prediction landscapes, employ statistical methods to outline the anticipated capabilities of the data processing system given particular data nuances. The creation of these predictive curves can be steered by algorithms adept in identifying and interpreting trends and patterns in the synthetic OOD data, a compilation designed to mirror a broad spectrum of potential real-world circumstances. This mechanism encourages continual refinement, as it allows for the incorporation of actual performance feedback into the system, thereby fine-tuning the predictive precision and dependability of the auxiliary machine learning model over time.

In optimizing the auxiliary machine learning model's performance and reliability, the management of the loss function or objective function can be important. It serves as a mathematical method to quantify the discrepancy between the model's predictions and actual outcomes, acting as a linchpin in gauging the assurance level that can be extended to various case scenarios.

Having precise control over the loss function or objective function of the auxiliary machine learning model, enables the fine-tuning of the model by adjusting various parameters and weights during the training phase, thereby channeling it to focus more on certain critical aspects pertinent to individual cases. In some examples, this function is manipulated in order to steer the model towards providing guarantees that are both realistic and aligned with the specific performance metrics established for different hospital environments.

The incorporation of synthetic OOD data aids in preparing the model to better handle a diverse range of scenarios, potentially enhancing its robustness and resilience to fluctuations in data quality and characteristics.

Optionally, synthetic OOD data can be generated using generative AI models such as diffusion models, Generative Adversarial Networks (GANs), and autoregressive models (e.g. PixelCNN) to generate medical images, transformerbased models to generate medical reports and EHR data, Recursive Neural Networks (RNNs) for the generation of time-series data, and Variational Autoencoders (VAEs) to generate new data similar to the training data.

Optionally, the transformations include performing corruptions, perturbations and/or augmentations on the in-distribution data used for training and evaluation of the primary machine learning model.

To create the synthetic OOD data, the system may be configured to modify the in-distribution data through techniques like data corruptions, perturbations, and augmentations. These transformations aim to make the auxiliary model robust and prepared for diverse data scenarios.

By applying various transformations such as corruptions, perturbations, and augmentations to the in-distribution data, the system can create a more comprehensive and diversified training dataset. This broader dataset significantly improves the system's ability to generalize and perform well even on unseen or slightly anomalous data.

Optionally, the transformations are applied with different severity levels.

These transformations encompass corruptions, perturbations, and augmentations, systematically applied at varying intensities or severity levels to mimic potential alterations the data may undergo in real-world scenarios. For instance, a matrix might be devised to apply transformations that progressively alter the sharpness of a photo or modulate other parameters at increasing rates. The system can thus generate a spectrum of synthetic data, from mildly transformed to heavily distorted.

Applying transformations at different severity levels ensures that the model is exposed to a wide spectrum of data variations during training. This exposure enhances the model's generalization capabilities, making it adept at handling various data conditions, thereby improving the predictive accuracy and reliability.

In the context of creating synthetic out-of-distribution (OOD) data for training the auxiliary machine learning model, utilizing perturbations and augmentations can serve as an important methodology. The process involves introducing various forms of corruptions into the images, such as embedding dead pixels or generating JPEG artifacts, to mimic potential real-world deviations in the data. The nature and extent of these corruptions are carefully chosen based on their relevance to the specific application. For instance, in a hospital setting where high-resolution imaging systems are utilized, JPEG artifacts, which result from heavy compression, are less likely to occur compared to handheld mobile devices where such distortions might be commonplace. Therefore, it can be important to select and apply a comprehensive range of perturbations and augmentations, encompassing all conceivable variations and disruptions that might arise in the particular application environment, to prepare the auxiliary machine learning model to function optimally in diverse conditions. This helps in enhancing the robustness and adaptability of the model, ensuring its reliable performance even in the presence of unexpected data variations.

Optionally, the uncertainty module includes a plurality of uncertainty quantification techniques, and/or the OOD module includes a plurality of OOD detection techniques, wherein the system is configured such that each of the techniques is selectively applicable based on the specific healthcare application area of the system.

The uncertainty module can be configured to calculate the uncertainty associated with each prediction made by the primary machine learning model using techniques such as measuring the distance between sigmoid outputs and a set cut-off value or implementing a Monte Carlo Dropout method, a deep ensemble, using Bayesian methods, bootstrap aggregating (Bagging), or quantile regression. Various other techniques may be employed.

Depending on the specific healthcare application in question, the system offers a choice among multiple uncertainty quantification and OOD detection techniques. This flexibility ensures that the system is optimized for the specific needs of a given healthcare scenario. With other words, the system can be easily customizable based on the application area, in an advantageous way.

The system's flexibility to selectively apply different techniques based on the specific healthcare application allows for customization and optimization of the system's performance in various domains, achieving higher efficiency and accuracy in each specific application.

Optionally, the system includes a configuration management module configured to evaluate the effectiveness of the various uncertainty quantification techniques and OOD detection techniques based on the specific healthcare application at hand, and to deactivate or remove the use of selected techniques that are determined to contribute insignificantly to the performance level for the particular healthcare application.

The configuration management module may be employed to optimize system performance by assessing the various uncertainty and OOD techniques. Ineffective or redundant techniques can be deactivated or removed, allowing the system to operate more efficiently in a specific healthcare application.

In this way, the system can effectively focus on the optimization and enhancement of computational efficiency through the selective use of metrics and techniques that are most relevant and reliable for specific healthcare application scenarios.

In some examples, the effectiveness of various techniques (including OOD techniques) is evaluated, and the system is configured to selectively deactivate less significant techniques based on the specific healthcare application. Thus, unnecessary computations can be avoided, enhancing efficiency by focusing on metrics with significant weightage.

The inclusion of a configuration management module allows the system to adjust its strategy by evaluating and selecting the most effective techniques for the specific healthcare application at hand. This optimization results in a system that can be operated at a higher efficiency, thus saving resources and time.

For example, in developing a data processing system for the detection of cancer, it is important to optimize computational efficiency by pinpointing the techniques that yield the most reliable results. For instance, in evaluating the effectiveness of various OOD techniques, the goal is to identify those which perform best for the application at hand. The auxiliary machine learning model, which is central to this process, assigns varying weights to different metrics based on their relevance and reliability for the specific application scenario. By focusing only on the metrics that have significant weightage, the system avoids unnecessary computations, thereby enhancing efficiency. This approach ensures that the model is not burdened with calculating metrics that do not contribute substantially to its performance, thus streamlining the process and facilitating quicker, yet accurate, decision-making in critical healthcare applications.

Optionally, the auxiliary machine learning model is configured to further receive as input the predictions generated by the primary machine learning model.

The auxiliary machine learning model can also use the predictions from the primary model as an input. This can aid in its assessment of the primary model's performance.

The auxiliary model's ability to receive the predictions of the primary model as inputs provides a more in-depth verification process. This process can find nuanced inconsistencies or errors, enhancing the overall reliability of the predictions generated by the system.

Optionally, the system further includes a data quality assessment module configured to quantify a quality of the patient data using one or more data quality metrics.

A distinct module is thus provided within the system which quantifies the quality of the patient data. This assessment ensures that the system's predictions are grounded in high-quality, reliable data. Including a data quality assessment module helps in maintaining the standard of input data, which can be important for achieving accurate and reliable predictions. This module may be used to ensure that only quality data feeds into the system, thus mitigating the risk of inaccurate predictions.

Optionally, the data quality metrics include at least one of: image sharpness, image contrast, image brightness, signal-to-noise ratio, noise level consistency, temporal resolution, data completeness, and data consistency.

The data quality assessment module can be configured to quantify the quality of patient data utilizing metrics such as image sharpness, contrast, and signal-to-noise ratio, enabling the system to anticipate potential performance drops due to data quality deterioration.

The system can measure data quality using various metrics, focusing specifically on image data. Metrics like image sharpness, contrast, brightness, and signal-to-noise ratio are considered, ensuring a comprehensive quality assessment. Utilizing detailed metrics for data quality analysis allows the system to make nuanced assessments of data quality, enhancing the reliability of the predictions generated based on this data. This detailed analysis can prevent errors and improve the overall effectiveness of the healthcare application.

Optionally, the uncertainty module is configured to use one or more techniques to quantify the uncertainty of the predictions from the primary machine learning model, said techniques including at least one of: measuring the distance between sigmoid output and a chosen cut-off value, deploying a deep ensemble technique, or implementing a Monte Carlo Dropout method.

The uncertainty module can employ diverse techniques to measure prediction uncertainty.

Optionally, the OOD module is configured to use one or more techniques to identify out of distribution data, said techniques including at least one of: reconstruction based methods such as Variational Auto Encoder (VAE) or MoodCat, training OOD detectors with supervised learning, using self-supervised learning with an auxiliary task (e.g. detect image orientation), or using the distance (e.g. Mahalanobis distance) to all class centroids.

Various reconstruction based methods can be used. The anomaly detection can be improved through the reconstruction of input data. Utilizing reconstruction-based methods like VAE (Variational Autoencoder) and MoodCat allows the system to identify OOD data by attempting to reconstruct the input data. These methods analyze the reconstruction error; a higher error generally indicates OOD data as the model struggles to accurately reconstruct data that is significantly different from the training data. This mechanism enables a more nuanced detection of OOD data, increasing the reliability of the system by avoiding errors or inconsistencies that might arise from undetected OOD data.

Training OOD detectors with supervised learning can improve identification of OOD data through training with labeled datasets. This technique may involve training the OOD detectors using supervised learning methods where the detectors have access to labeled datasets. The labeled datasets help the model to clearly demarcate the boundaries between in-distribution and out-of-distribution data. The training process fine-tunes the detectors to be adept at distinguishing between in-distribution and OOD data, hence minimizing false alarms and improving the accuracy and reliability of the system.

Using distance metrics (e.g., Mahalanobis distance) to analyze distance to class centroids can enhance classification and identification of OOD data. Implementing distance metrics like Mahalanobis distance enables the system to evaluate the proximity of a data point to the centroids of known classes (in-distribution data). If the data point lies at a significant distance from all known class centroids, it is classified as OOD. This method provides a geometric approach to identifying OOD data, making the system robust against potential anomalies that might skew the prediction results.

Optionally, the system includes a calibration module configured to calibrate the uncertainty of the auxiliary machine learning model, using techniques such as Temperature Scaling, Platt Scaling, isotonic regression, beta calibration, or Dirichlet calibration.

The calibration module can be configured to adjust the uncertainty levels of the auxiliary machine learning model through techniques such as Temperature Scaling, Platt Scaling, isotonic regression, beta calibration, or Dirichlet calibration, ensuring a finely-tuned response to varying data inputs. By employing the calibration module within the system, the auxiliary machine learning model is able to make an estimation of the obtained accuracy of the primary ML model in the clinical practice.

Temperature scaling can be used to adjust confidence levels of model predictions for better reliability and trustworthiness. Temperature scaling can be a post-processing method that adjusts the softmax outputs of the model (or confidence levels) using a learned temperature parameter. This process helps in calibrating the predicted probabilities to be more aligned with the true probabilities. Essentially, it fine-tunes the confidence levels of the predictions, avoiding overly confident incorrect predictions and under-confident correct predictions. This modification can lead to more reliable and realistic uncertainty estimates, which can aid in making more informed healthcare decisions.

Platt Scaling can be used for conversion of model scores to probabilities that are well-calibrated and aligned with the true outcome distribution. Platt scaling is another post-processing calibration technique that transforms the output scores of the model into probabilities using a sigmoid function. This method is particularly effective in scenarios where the output scores are not well-calibrated to represent probabilities, as it brings the predicted probabilities closer to the true probabilities by fitting the scores to a logistic regression model. This adjustment can enhance the reliability of the predictions and foster trust in the decisions derived from the model's outputs.

Advantageously, through the refined calibration of uncertainty estimates, a more nuanced and informed decision-making can be achieved, potentially leading to better healthcare outcomes and more effective resource allocation.

Optionally, the system is deployable across various medical settings, said settings comprising different clinical practices or different sites within the same clinical practice, wherein each deployment has the same primary machine learning model.

The system can be implemented in various medical environments, including different hospitals or even different departments within the same hospital. Despite these varied deployments, each instance uses the same primary machine learning model.

The ability to deploy the system across various medical settings while maintaining a consistent primary machine learning model ensures a uniform standard of predictions across different locations, promoting standardized, high-quality healthcare decision support irrespective of the site of deployment.

Optionally, the auxiliary machine learning model has the output of the primary machine learning model (i.e. the prediction of the primary machine learning model) as input. Advantageously, this allows the model to use more application knowledge (e.g. image quality may be less important when clear signs of disease are present).

According to an aspect, the invention provides for a data-driven decision support method for healthcare applications, implemented in a data processing system, the method comprising: receiving patient data for processing in a primary machine learning model; generating predictions based on the patient data using the primary machine learning model; calculating uncertainty for each prediction generated by the primary machine learning model using an uncertainty module; assessing whether the patient data is in-distribution or out-of-distribution using an out-of-distribution (OOD) module, the OOD module incorporating multiple OOD detectors; receiving inputs from the uncertainty module and OOD module in a trained auxiliary machine learning model; outputting a value from the auxiliary machine learning model, the value indicative for which cases a predetermined target performance level for predictions generated by the primary machine learning model is met.

During the operation, the data processing system receives patient data, which is then processed by the primary machine learning model to generate predictions. Simultaneously, the uncertainty for each prediction is calculated, and the data distribution type (ID or OOD) is assessed. This information is then channeled into the auxiliary machine learning model, which outputs a value indicative of the adherence to the predetermined performance levels.

Advantageously, in specific medical settings, the auxiliary machine learning model can act as a guaranteed performance model that affirms that the claimed performance levels are being met at any given time, accounting for various data complexities and quality factors. It is built to adapt and respond to changing data conditions, offering a reliable and technically robust solution to uphold the accuracy claims made by artificial intelligence or machine learning data processing products.

In order to effectively evaluate and apply the auxiliary machine learning model (cf. guaranteed performance, GP, model) across various medical settings, specific metrics may be used. Firstly, assurance metrics may be used to determine the confidence level of the model's predictions, establishing its reliability. Secondly, OOD metrics are utilized in an advantageous way to discern whether the data being analyzed aligns with in-distribution (ID) or out-of-distribution (OOD) criteria, thereby ensuring that predictions are made on pertinent data.

In some examples, data quality metrics are also used, offering insights into the integrity and clarity of the patient data, potentially identifying reasons for performance deviations. A spectrum of techniques can be used during the training phase of the auxiliary machine learning model, so that for any specific application, the most effective metrics are employed to maximize confidence, accurately identify OOD data, and assess data quality.

The method allows not only to assess if the promised performance metrics are being met for a particular dataset but to also provides for refined, real-time performance estimates specific to different operational environments such as individual hospitals. This may involve a multi-faceted analysis where the auxiliary machine learning model meticulously evaluates various data streams in real-time, segmenting them into distinct series or batches, such as patient series A and patient series B. Through continuous monitoring and assessment, employing a complex array of assurance metrics, OOD metrics, and data quality metrics, the system can dynamically gauge the current performance level.

For instance, if an artificial intelligence product promises an accuracy of e.g. 90%, the system can validate if this claim holds true for patient series A while concurrently indicating that it might not be applicable for patient series B, possibly due to varying data quality or other environmental factors. This granularity in performance evaluation is taken a step further by extrapolating the analyzed data to generate predictive performance estimates for specific time frames. This means that in a particular clinical setting or hospital, the system might predict an overall performance accuracy of e.g. 82% based on current and historical data analysis. Consequently, it equips medical professionals with nuanced insights, enabling them to make informed decisions on whether to rely on the system's predictions or to intervene based on the fluctuating performance metrics, thereby fostering a more responsive and adaptive healthcare delivery model. This mechanism of operation not only ensures reliability but enhances the transparency and accountability of artificial intelligence applications in healthcare settings.

The auxiliary machine learning model can operate with a nuanced discernment mechanism, actively differentiating between instances where it can unequivocally assure high-level performance and instances where the performance might be compromised due to various factors such as data anomalies, sub-par data quality, or a disparity between the existing data set and new data parameters (like different ethnicity factors that were not prevalent during the product's creation phase). This intelligent stratification of cases, underpinned by deep analysis and predictive algorithms, facilitates a more targeted and efficient approach to medical evaluations. For example, in situations where the model predicts a less favorable performance, it signals the necessity for a manual re-evaluation or a more detailed analysis by the healthcare provider. This preemptive identification of potential pitfalls not only enhances the reliability of the artificial intelligence data processing system, but also serves as an important tool for medical practitioners, aiding them in making informed decisions on when to augment the artificial intelligence's analysis with human expertise.

According to an aspect, the invention provides for a system comprising a plurality of distributed data processing systems according to the disclosure, deployed across various medical settings for providing data-driven decision support, wherein the medical settings comprise different clinical practices or different sites within a same clinical practice, and wherein each subsystem utilizes, or has access to, the same primary machine learning model.

The system can be scaled and deployed across various medical settings, from different hospitals to different sites within a single hospital. Despite the geographical differences, each subsystem shares or has access to the same primary machine learning model, ensuring consistent predictive performance.

According to an aspect, the invention provides for a system for predictive medical analysis. The system may have an artificial intelligence module configured for deployment in a plurality of settings for performing predictive analysis in medical applications, wherein for each patient examination the AI module generates a prediction based on patient data; an uncertainty module communicatively coupled with the AI module, configured to calculate a measure of uncertainty for the prediction generated by the AI module; an Out-of-Distribution (OOD) module communicatively coupled with the AI module, comprising a plurality of OOD detectors, wherein the OOD module is configured to analyze the patient data and determine whether said patient data is in-distribution (ID) or out-of-distribution (OOD) based on a predetermined criteria; and an auxiliary machine learning (ML) model communicatively coupled with the uncertainty module and the OOD module, configured to receive as input the measure of uncertainty from the uncertainty module and the determination of the patient data being ID or OOD from the OOD module, wherein the auxiliary ML model is trained to output a value indicative of whether a predetermined target performance level for the prediction generated by the AI module is reached.

Advantageously, the Al module, the uncertainty module, the OOD module, and the auxiliary ML model operate in coordination to provide enhanced predictive analysis for medical applications in a variety of deployment settings.

It will be appreciated that any of the aspects, features and options described in view of the data processing system apply equally to the data-driven decision support method and the described system with distributed data processing systems. It will also be clear that any one or more of the above aspects, features and options can be combined.

### BRIEF DESCRIPTION OF THE DRAWING

The invention will further be elucidated on the basis of exemplary embodiments which are represented in a drawing. The exemplary embodiments are given by way of non-limitative illustration. It is noted that the figures are only schematic representations of embodiments of the invention that are given by way of non-limiting example.

In the drawing:
Fig. 1 shows a schematic diagram of an embodiment of a data processing system;
Fig. 2 shows a schematic diagram of an embodiment of a data processing system;
Fig. 3 shows a schematic diagram of an embodiment of a data processing system; and
Fig. 4 shows a schematic diagram of an embodiment of a method.

### DETAILED DESCRIPTION

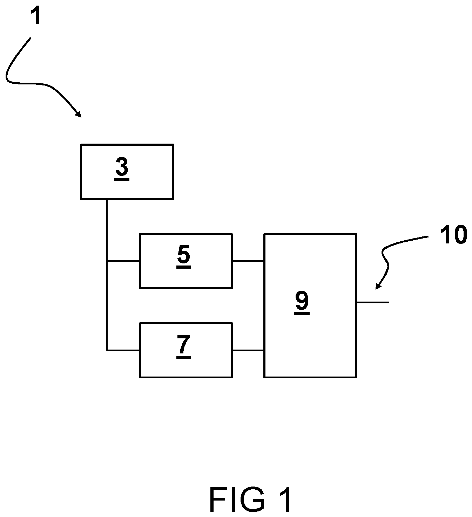

Fig. 1 shows a schematic diagram of an embodiment of a data processing system 1 for providing data-driven decision support in healthcare applications. The data processing system 1 comprises a primary machine learning model 3 configured to generate predictions based on patient data. The system 1 also includes an uncertainty module 5, linked to the primary machine learning model 3, configured to calculate uncertainty for each prediction of the primary machine learning model. Furthermore, the system 1 comprises an out-of-distribution, OOD, module 7, incorporating an OOD detector, wherein the OOD module is configured to assess whether the patient data is in-distribution or out-of-distribution. The system 1 also includes an auxiliary machine learning model 9, trained to receive at least inputs from the uncertainty module 5 and OOD module 7, and based on the inputs to output 10 a value indicative of whether a predetermined target performance level for predictions generated by the primary machine learning model 3 is met.

The uncertainty module 5 calculates how certain the predictions of the primary machine learning model 3 are. The OOD 7 Module determines whether the patient data is within the expected distribution (in-distribution) or not (out-of-distribution). This module analyses the patient data to identify if it falls within the known distribution (patterns it has seen during training) or if it is an anomaly (a pattern it has not seen before), thus helping in recognizing unusual cases or data errors. The auxiliary machine learning 9 model evaluates the performance level of the predictions generated by the primary model. This secondary machine learning model receives inputs from both the uncertainty module 5 and the OOD module 7. The auxiliary machine learning model 9 acts as a "guaranteed performance" (GP) model and analyzes these inputs to ascertain if the predictions made by the primary model meet a certain standard of performance. If the predictions are within an acceptable range of uncertainty and are based on in-distribution data, the auxiliary model would indicate that the performance level is met.

In some examples, the data processing system 1 starts by receiving patient data. The primary machine learning model 3 processes this data to generate initial predictions. Subsequently or simultaneously, an uncertainty analysis and data distribution analysis can be performed. The uncertainty module 5 calculates how "uncertain" or "confident" the system 1 is about each of these predictions. The OOD module 7 evaluates the data to identify if it is within a recognized distribution or not, to note any potential anomalies. The auxiliary machine learning model 9 then takes inputs from both the uncertainty module 5 and the OOD module 7 to evaluate if the predictions from the primary model meet predetermined performance level or not. The combination of primary and auxiliary machine learning models 3, 9 enables to substantiate the accuracy claims across different settings.

Out-of-Distribution (OOD) data - data that deviates significantly from the data utilized during the machine learning's training and evaluation phase, is identified and effectively handled. The system can better guarantee the promised performance levels under specified conditions and clearly indicate or communicate the circumstances where the guarantee is not applicable.

In some examples, to address the limitations of pre-deployment OOD data availability, synthetic OOD data is generated. This data emulates possible variations and alterations that real data might undergo, providing a rich basis for product development. The process involves introducing incremental alterations, indexed by severity levels, to In-Distribution (ID) data - data available during the primary machine learning model development and evaluation phase. Factors such as image sharpness and adjustment degree can be modulated to generate a diverse dataset. Additionally or alternatively, generative AI techniques are used.

The auxiliary machine learning model can be used to validate the accuracy claims made by the system/product under various conditions, accounting for potential data alterations and variations in different medical settings. In some examples, it can utilize synthetic OOD data, generated with various perturbations and corruptions pertinent to specific applications, to evaluate and guarantee the performance of the AI system/product under those conditions.

In some examples, to simulate real-world scenarios more closely, the auxiliary machine learning model introduces relevant data perturbations and augmentations, such as dead pixels or JPEG artifacts, based on the specific application environment. For instance, in hospital settings where high-quality images are a norm, compression artifacts might be less relevant, thus guiding the type of perturbations to be applied during synthetic OOD data creation.

The auxiliary machine learning model 9 can be utilized to affirm that the AI system/product meets the promised performance levels under designated conditions in various medical settings. Furthermore, it can provide performance estimates for specific data sets within particular time frames, offering detailed insights into the expected efficacy and aiding medical professionals in making informed decisions. It delineates cases where performance guarantees are upheld from those where they might not be, facilitating necessary interventions.

The system can take into account the inherent difficulty level associated with different cases and the impact of data quality on system performance. Factors like image sharpness, contrast, and signal-to-noise ratio are accounted for, with the understanding that data quality might influence the reliability of the performance claims.

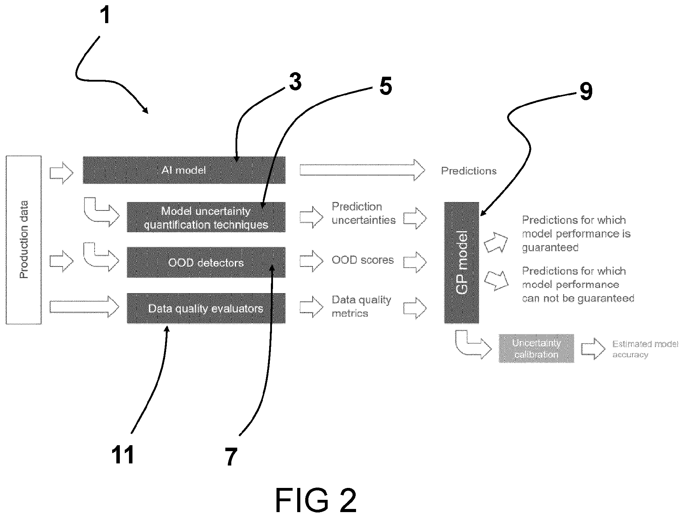

Fig. 2 shows a schematic diagram of an embodiment of a data processing system 1 during model deployment. The system 1 includes a primary machine learning model 3 (cf. Al model), an uncertainty module 5 and an OOD module 7. The system 1 also includes optionally a data quality assessment module 11 configured to quantify a quality of the patient data using one or more data quality metrics. Further, the outputs of the uncertainty module 5, OOD module 7 and the data quality assessment module 11 are provided as input to the auxiliary machine learning model 9 (cf. guaranteed performance, GP, model).

The system improves the determination of which performance the model obtains in a wide variety of settings (e.g. clinical practice such as hospital) when only model performance is known on historic data of one setting (e.g. clinical practice), or a limited number of settings (e.g. clinical practices). In this way, professionals or practitioners are able to more robustly trust that the system will obtain the promised performance level (e.g. F1 score of 90%) in their clinical practice, on their patients, with their specific/custom devices, such as imaging or measurement devices. In the case that the AI system is not able to reach the wanted performance level (e.g. due to bad data quality or usage of an unsupported imaging device), it becomes possible to inform of this in a timely manner. This is in contrast with known solutions in the art that are unable to guarantee that their system will perform well for example in each hospital at each moment in time. Furthermore, they currently have to rely on their customers to inform them if and when underperformance of their device is observed.

Three main causes for deterioration in model performance due to changes to the data distribution can be identified, namely:
- The presence of many difficult cases. Some cases are difficult to analyze, both for humans and for the AI. (e.g. trying to determine the correct diagnose on a borderline case) No high performance can be expected on those cases.
- The presence of Out Of Distribution (OOD) data. OOD data is data which differs from the data used to train and evaluate the AI model. This difference can, among others, be due to differences in the patient demographics (e.g. age, ethnicity, sex), properties of the imaging or measurement device used (e.g. device model, used device settings, presence of dust on lens), the environment in which the data was collected (e.g. taking pictures in a windowless examination room, versus in sunny room), and the presence of different comorbidities (e.g. when the AI is used in a COPD clinic).
- Low data quality (e.g. unsharp medical images, or time series with low signal noise ratio) can reduce the performance of both human professionals and of automated AI systems analyzing the data.

The uncertainty of the predictions of the primary machine learning model 3 can be quantified using one or more techniques, using for example distance between sigmoid output and chosen cut-off value, employing deep ensemble method, Monte Carlo Dropout method, etc.

The model input data can be sent through a diverse collection of Out Of Distribution (OOD) detectors, e.g. using the reconstruction error of a Variational Auto Encoder (VAE), the usage of self-supervised learning techniques, e.g. the addition of an auxiliary task to predict the orientation of the picture, or using Mahalanobis Distance on the activations of the pen-ultimate network layer. The quality of the model input data can be quantified with various data quality metrics, e.g. image sharpness, image contrast, image brightness, and signal noise ratio of time series data.

Advantageously, an auxiliary machine learning model (e.g. a neural network model) is trained. This model can be called the Guaranteed Performance (GP) model, which predicts for each case if there is sufficient confidence whether the primary machine learning model prediction is correct or not. The inputs of this auxiliary machine learning model are for each case the model uncertainty predictions, the outputs of the OOD detectors, and optionally also the data quality metrics. Synthetic OOD data is created from the ID data used for the training and evaluation of the auxiliary machine learning model. The synthetic OOD data is created by applying a wide range of corruptions, perturbations and augmentations (e.g. color changes, zooming, cropping, elastic deformations, the addition of noise, addition of jpg artifacts, changing image brightness levels). In this example, they are applied with different severity levels.

The GP model is trained using both the ID data and the synthetically generated OOD data.

The loss function used during the training of the GP model compares the output of the GP model (prediction for which model performance is guaranteed or not) with the fact that the original primary machine learning model prediction was considered correct or not. Note that with 'correct', it is meant correct within the specifications of the machine learning model. For instance, a predictive algorithm that generated an output marginally different from the accurate value might still be deemed acceptable within the parameters of the computational model. An example of a loss function which could be used is the binary cross entropy function. Typically a higher weight will be given to false positives (the system incorrectly identifies a result as accurate; the auxiliary machine learning model assesses that the prediction of the primary machine learning model is correct while it is not) compared to false negatives (the system incorrectly labels an accurate result as inaccurate; the auxiliary machine learning model assesses that the prediction of the primary machine learning model is not correct while it is). The choice of these weights will depend on how critical the correctness of the model output is for the considered medical application.

Additionally, the uncertainty of the auxiliary machine learning model can be predicted (e.g. with Temperature Scaling, Platt Scaling, isotonic regression, beta calibration, or Dirichlet calibration). In this way, it becomes possible to correctly estimate the accuracy of the model prediction.

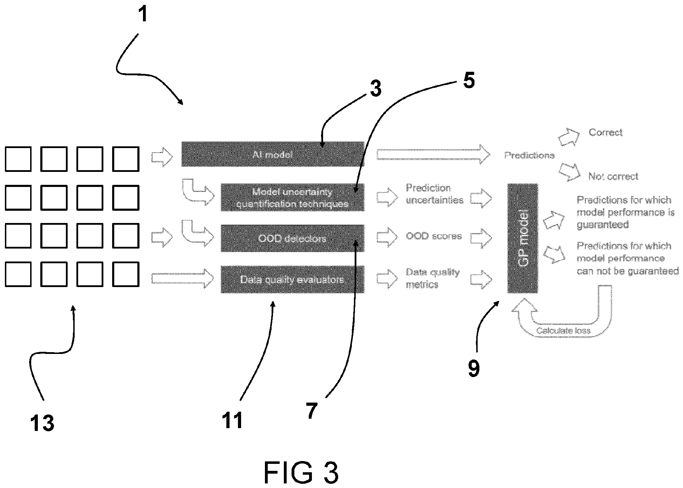

Fig. 3 shows a schematic diagram of an embodiment of a data processing system 1 during training of the auxiliary machine learning model 9. Synthetic OOD data is created by applying a wide variety of relevant corruptions and perturbations on the ID data of the primary machine learning model 3.

In this example, the employed auxiliary machine learning model 9 is trained using both in-distribution data and synthetic OOD data that is created with the help of a generative Al model, or by applying a plurality of transformations 13 to the in-distribution data used for training and evaluation of the primary machine learning model 3. The transformations include performing corruptions, perturbations and/or augmentations on the in-distribution data used for training and evaluation of the primary machine learning model 3. The transformations can be applied with different severity levels.

Advantageously, the user can be confident that the data-driven system will reach the promised performance level for all cases for which the auxiliary machine learning model has indicated that it is confident that the prediction of the primary machine learning model is correct. This is the case for all model performance metrics which are independent of the class prevalence (e.g. sensitivity, specificity, Positive Likelihood Ratio (PLR), Negative Likelihood Ratio (NLR), F1 score). The F1 score is a metric in machine learning for evaluating the performance of a binary classification model. It is a harmonic mean of the precision and recall of the model, and it provides a single score that balances both measures.

Note that the advantageous system can be applied to a wide application area (e.g. medical image analysis classification models, regression models, segmentation model, time series analysis) where the difference in importance of false positives and false negatives is application dependent. To offer support for a wide range of application areas, the system can employ a wide range of techniques to quantify model uncertainty, detect OOD cases, and optionally to evaluate the data quality. This is because the optimal technique for one application might not be able to produce satisfactory results for another application.

In some examples, the proposed solution can be made more computational efficient by removing the components which do not significantly contribute for the application at hand (e.g. a VAE might be a poor choice of OOD detector for some medical image analysis applications).

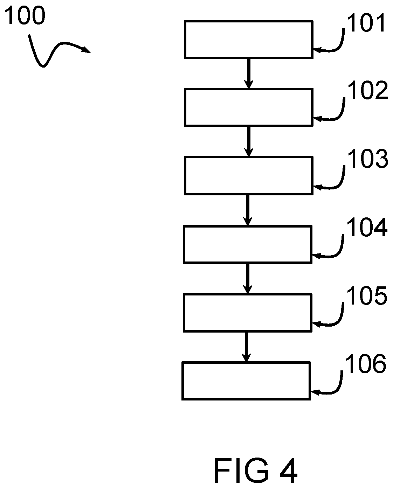

Fig. 4 shows a schematic diagram of an embodiment of a data-driven decision support method 100 for healthcare applications, implemented in a data processing system 1. In a first step 101, patient data for processing in a primary machine learning model is received. In a second step 102, predictions based on the patient data using the primary machine learning model are generated. In a third step 103, uncertainty for each prediction generated by the primary machine learning model using an uncertainty module is calculated. In a fourth step 104, it is assessed whether the patient data is in-distribution or out-of-distribution using an out-of-distribution (OOD) module, the OOD module incorporating multiple OOD detectors. In a fifth step 105, inputs from the uncertainty module and OOD module are received in a trained auxiliary machine learning model. In a sixth step 106, a value is outputted by the auxiliary machine learning model, the value indicative of whether a predetermined target performance level for predictions generated by the primary machine learning model is met.

It will be appreciated that in the context of artificial intelligence, particularly in machine learning and deep learning models, the terms "in-distribution data" and "out-of-distribution data" refer to whether the data that is dealt with is within the same distribution that the model was trained on, or not. In-distribution data refers to data that comes from the same distribution as the training set. For example, if a machine learning model is trained to classify images of X and Y, and new images of X and Y that it has never seen before are provided as input, these new images would be considered in-distribution. This is because they are drawn from the same population and have similar characteristics to the images the model was trained on. Out-of-distribution data, on the other hand, comes from a different distribution than the one the model was trained on. Using the same example, if the X-Y classifier is shown an image of Z, this would be considered out-of-distribution data. The model has never seen a Z during training, and therefore, this data falls outside of the model's training distribution. The ability of a model to handle out-of-distribution data is a significant factor in determining its robustness and generalizability. Models trained only on in-distribution data may perform poorly when faced with out-of-distribution data, which can be a major issue in real-world applications where the data may not always perfectly match the training distribution.

It will be appreciated that the method may include computer implemented steps. All above mentioned steps can be computer implemented steps. Embodiments may comprise computer apparatus, wherein processes performed in computer apparatus. The invention also extends to computer programs, particularly computer programs on or in a carrier, adapted for putting the invention into practice. The program may be in the form of source or object code or in any other form suitable for use in the implementation of the processes according to the invention. The carrier may be any entity or device capable of carrying the program. For example, the carrier may comprise a storage medium, such as a ROM, for example a semiconductor ROM or hard disk. Further, the carrier may be a transmissible carrier such as an electrical or optical signal which may be conveyed via electrical or optical cable or by radio or other means, e.g. via the internet or cloud.

Some embodiments may be implemented, for example, using a machine or tangible computer-readable medium or article which may store an instruction or a set of instructions that, if executed by a machine, may cause the machine to perform a method and/or operations in accordance with the embodiments.

Various embodiments may be implemented using hardware elements, software elements, or a combination of both. Examples of hardware elements may include processors, microprocessors, circuits, application specific integrated circuits (ASIC), programmable logic devices (PLD), digital signal processors (DSP), field programmable gate array (FPGA), logic gates, registers, semiconductor device, microchips, chip sets, et cetera. Examples of software may include software components, programs, applications, computer programs, application programs, system programs, machine programs, operating system software, mobile apps, middleware, firmware, software modules, routines, subroutines, functions, computer implemented methods, procedures, software interfaces, application program interfaces (API), methods, instruction sets, computing code, computer code, et cetera.

Herein, the invention is described with reference to specific examples of embodiments of the invention. It will, however, be evident that various modifications, variations, alternatives and changes may be made therein, without departing from the essence of the invention. For the purpose of clarity and a concise description features are described herein as part of the same or separate embodiments, however, alternative embodiments having combinations of all or some of the features described in these separate embodiments are also envisaged and understood to fall within the framework of the invention as outlined by the claims. The specifications, figures and examples are, accordingly, to be regarded in an illustrative sense rather than in a restrictive sense. The invention is intended to embrace all alternatives, modifications and variations which fall within the scope of the appended claims. Further, many of the elements that are described are functional entities that may be implemented as discrete or distributed components or in conjunction with other components, in any suitable combination and location.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality. The term "and/or" includes any and all combinations of one or more of the associated listed items. The mere fact that certain measures are recited in mutually different claims does not indicate that a combination of these measures cannot be used to an advantage.

## Claims

1. A data processing system for providing data-driven decision support in healthcare applications, comprising:
a primary machine learning model configured to generate predictions based on patient data;
an uncertainty module, linked to the primary machine learning model, configured to calculate uncertainty for each prediction of the primary machine learning model;
an out-of-distribution (OOD) module, incorporating an OOD detector,
wherein the OOD module is configured to assess whether the patient data is in-distribution or out-of-distribution;
an auxiliary machine learning model, trained to receive at least inputs from the uncertainty module and OOD module, and based on the inputs to output a value indicative of which cases a predetermined target performance level for predictions generated by the primary machine learning model is met.

2. The data processing system according to claim 1, wherein the system further comprises an alarm module communicatively linked to the auxiliary machine learning model, configured to assess the value outputted by the auxiliary machine learning model, wherein the alarm module includes a performance monitoring algorithm designed to compare the value against the predetermined target performance level threshold and output an alarm signal via at least one user interface if the value falls below the predetermined target performance level threshold.

3. The data processing system according to claim 1 or 2, wherein the employed auxiliary machine learning model is trained using both in-distribution data and synthetic OOD data that is created by using a generative AI model, and/or by applying a plurality of transformations to the in-distribution data used for training and evaluation of the primary machine learning model.

4. The data processing system according to claim 3, wherein the transformations include performing corruptions, perturbations and/or augmentations on the in-distribution data used for training and evaluation of the primary machine learning model.

5. The data processing system according to claim 4, wherein the transformations are applied with different severity levels.

6. The data processing system according to any one of the preceding claims, wherein the uncertainty module includes a plurality of uncertainty quantification techniques, and/or the OOD module includes a plurality of OOD detection techniques, wherein the system is configured such that each of the techniques is selectively applicable based on the specific healthcare application area of the system.

7. The data processing system according to claim 6, wherein the system includes a configuration management module configured to evaluate the effectiveness of the various uncertainty quantification techniques and OOD detection techniques based on the specific healthcare application at hand, and to deactivate or remove the use of selected techniques that are determined to contribute insignificantly to the performance level for the particular healthcare application.

8. The data processing system according to any one of the preceding claims, wherein the auxiliary machine learning model is configured to further receive as input the predictions generated by the primary machine learning model.

9. The data processing system according to any one of the preceding claims, wherein the system further includes a data quality assessment module configured to quantify a quality of the patient data using one or more data quality metrics.

10. The data processing system according to claim 9, wherein the data quality metrics include at least one of: image sharpness, image contrast, image brightness, signal-to-noise ratio, noise level consistency, temporal resolution, data completeness, and data consistency.

11. The data processing system according to any one of the preceding claims, wherein the uncertainty module is configured to use one or more techniques to quantify the uncertainty of the predictions from the primary machine learning model, said techniques including at least one of: measuring the distance between sigmoid output and a chosen cut-off value, deploying a deep ensemble technique, implementing a Monte Carlo Dropout method, using Bayesian methods, bootstrap aggregating (Bagging), or quantile regression.

12. The data processing system according to any one of the preceding claims, wherein the OOD module is configured to use one or more techniques to identify out of distribution data, said techniques including at least one of: reconstruction based methods such as using the reconstruction based methods such as a Variational Auto Encoder, training OOD detectors with supervised learning, using self-supervised learning, or using the distance to all class centroids.

13. The data processing system according to any one of the preceding claims, wherein the system includes a calibration module configured to calibrate the uncertainty of the auxiliary machine learning model, using techniques such as Temperature Scaling, Platt Scaling, isotonic regression, beta calibration, or Dirichlet calibration.

14. The data processing system according to any one of the preceding claims, wherein the system is deployable across various medical settings, said settings comprising different clinical practices or different sites within a same clinical practice, wherein each deployment has a same primary machine learning model.

15. A data-driven decision support method for healthcare applications, implemented in a data processing system, the method comprising:
receiving patient data for processing in a primary machine learning model;
generating predictions based on the patient data using the primary machine learning model;
calculating uncertainty for each prediction generated by the primary machine learning model using an uncertainty module;
assessing whether the patient data is in-distribution or out-of-distribution using an out-of-distribution (OOD) module, the OOD module incorporating multiple OOD detectors;
receiving inputs from the uncertainty module and OOD module in a trained auxiliary machine learning model;
outputting a value from the auxiliary machine learning model, the value indicative of which cases a predetermined target performance level for predictions generated by the primary machine learning model is met.

16. A system comprising a plurality of distributed data processing systems according to any one of the claims 1-14, deployed across various medical settings for providing data-driven decision support, wherein the medical settings comprise different clinical practices or different sites within a same clinical practice, and wherein each subsystem utilizes, or has access to, the same primary machine learning model.
